# EUROPEAN PATENT SPECIFICATION

(11) **EP 2 999 480 B1**
(45) Date of publication and mention of the grant of the patent: **04.09.2019**
(21) Application number: 13791901.5
(22) Date of filing: 09.10.2013
(51) Int. Cl.: A61K 36/88, A61P 25/28

(54) **EXTRACT OF HIPPEASTRUM PAPILIO RICH IN GALANTHAMINE**
EXTRAKT VON HIPPEASTRUM PAPILIO REICH AN GALANTHAMIN
EXTRAIT D'HIPPEASTRUM PAPILIO RICHE EN GALANTHAMINE

(30) Priority: 07.03.2013 BG 11142013
(43) Date of publication of application: 30.03.2016
(73) Proprietor: Berbee Beheer BV., 2161 LR Lisse (NL); Berkov, Strahil Hristov, 2050 Ihtiman (BG)
(72) Inventor: BERKOV, Strahil Hristov, 2050 Ihtiman (BG); ARMENGOL, Jaime Bastida, E-08850 Gava (ES); MAHRER, Carlos Codina, E-08028 Barcelona (ES); DE ANDRADE, Jean Paulo, 95043 - 420 Caxias do Sul Rio Grande do Sul (BR); BERBEE, Rudolf Leonardus Maria, NL-2215 RZ Voorhout (NL)
(74) Representative: van Dam, Vincent
(86) International application number: PCT/BG2013/000046
(87) International publication number: WO 2014/134692

(56) References cited:
- JEAN PAULO DE ANDRADE ET AL: "Alkaloids from Hippeastrum papilio", MOLECULES, vol. 16, no. 12, 18 August 2011 (2011-08-18), pages 7097-7104, XP055092003, DOI: 10.3390/molecules16087097
- STRAHIL BERKOV ET AL: "Development and validation of a GC-MS method for rapid determination of galanthamine in Leucojum aestivum and Narcissus ssp.: A metabolomic approach", TALANTA, vol. 83, no. 5, 1 February 2011 (2011-02-01), pages 1455-1465, XP055091989, ISSN: 0039-9140, DOI: 10.1016/j.talanta.2010.11.029
- EL MOHGAZI A M ET AL: "PHYTOCHEMICAL INVESTIGATION OF HIPPEASTRUM-VITTATUM GROWING IN EGYPT PART 2 ISOLATION AND IDENTIFICATION OF NEW ALKALOIDS", PLANTA MEDICA, vol. 28, no. 4, 1975, pages 336-342, XP009174890, ISSN: 0032-0943 cited in the application

## Description

### Field of the invention

The invention concerns the composition of extracts from *Hippeastrum papilio* and the use of their components for the production of pharmaceuticals and dietary supplements in pharmaceutical and food industries.

### Prior art

It is known from the state of the art that the species of the genus *Hippeastrum* (Amaryllidaceae) are large tropical plants that show significant variation in their alkaloid composition (Rev. Latinoamer. Quím. 2012, 40: 83). It is also known that there is a significant intraspecific variation in the composition and concentration of alkaloids in different species of the family Amaryllidaceae such as *Leucojum aestivum, Galanthus elwesii*, and *G. nivalis* (Chem. Biodiv. 2011, 8: 115; Biochem. Syst. Ecol., 2013, 46: 152), but no evidence of such a variation within species or cultivars of the genus *Hippeastrum* has been found.

From the state of the art the composition of an extract from the genus *Hippeastrum* is known to include the alkaloids: galanthamine, galanthine, lycorine, hippamine, narcissidine, hippeastrine, haemanthamine, crinidine, powelline and tazettine (Archiv der Pharmazie und Berichte der Deutschen Pharmazeutischen Gesellschaft 1962, 295: 920).

From the state of the art, the composition of an extract from the genus *Hippeastrum* is known to include the alkaloids: 2,7-dimethoxyhomolycorine, candimine, tazettine, pretazettine, epimacronine, haemanthamine, hamayne and triphaeridine (Magn. Res. Chem. 2011, 49: 668).

The composition of an extract from the genus *Hippeastrum* is also known to include the alkaloids: hippadine, hippacine, hippeastrine, vittatine, pancracine and tazettine (Mohgazi, et al., Planta Medica 1975, vol 28, no. 4 - pages 336-342).

De Andrade et al., Molecules, vol. 16, no. 12, pages 7097-7104 describes alkaloids from Hippeastrum papilio.

To the best of our knowledge, there are no data on the percentage ratio of the alkaloids in the alkaloid composition of extracts from the genus *Hippeastrum* measured by an analytical technique such as gas chromatography-mass spectrometry (GC-MS) or high-pressure liquid chromatography (HPLC).

There are no data in the literature on the industrial production of galanthamine from the genus *Hippeastrum.*

Galanthamine (chemical formula A) is a known, natural alkaloid produced by plants of the family Amaryllidaceae, including the relatively small plants of the genera *Galanthus*, *Narcissus*, *Leucojum* and *Lycoris* (Biotechnol. & Biotechnol. Eq. 2009: 1170). Galanthamine is a reversible cholinesterase inhibitor (AChE) that has analgesic and antioxidant properties. It is used to treat Alzheimer's disease (Drugs 2000, 60: 1095) and dependence on alcohol, drugs and nicotine (US Pat. 5,643,905). WO/2010/025485 patent describes the use of galanthamine hydrobromide to produce drugs to treat glaucoma. EP0236684 patent describes the use of galanthamine or its analogues for the treatment of Alzheimer's disease. According to patent WO/2006/099635, galanthamine is isolated from biological material, preferably from plants of the genus *Galanthus*, *Narcissus*, *Leucojum* and/or *Lycoris.*

It is also known that dietary supplements containing galanthamine, and in particular *Leucojum aestivum* extract, are used to improve muscle strength and endurance in athletes (WO2000041540).

Narwedine (chemical forula B) is another known reversible cholinesterase inhibitor (Proteins 2001, 42: 182), used as a precursor for the synthesis of galanthamine (WO2005/030713).

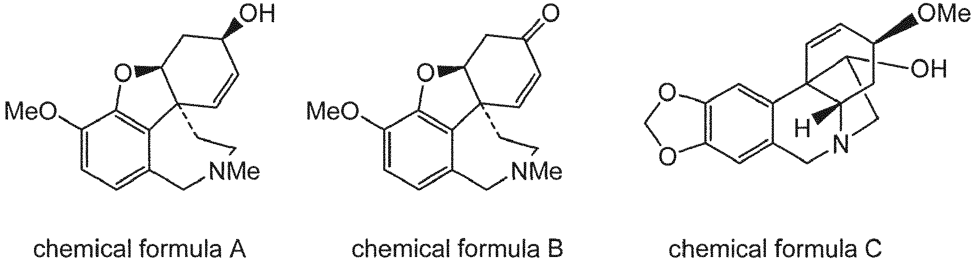

Haemanthamine (chemical formula C) is another known alkaloid. This lead molecule has been shown to be a potent inducer of apoptosis in tumor cells at micromolar concentrations (Phytochemistry 2007, 68: 1068), and consequently has been the subject of intense research in recent years.

The disadvantages of the known compositions obtained from plant sources of galanthamine, described in patent WO/2006/099635, are that they contain significant amounts of accompanying alkaloids that influence its isolation, and/or have toxic effects, so the direct use of extracts from these plants for the preparation of pharmaceuticals and dietary supplements is undesirable. For example, in different populations of *Leucojum aestivum,* galanthamine ranges from 0.03 to 0.4 % of the dry weight, and is accompanied by substantial amounts of lycorine, homolycorine and 8-O-demethylhomolycorine (Biochem. Syst. Ecol., 2013, 46: 152). The *Narcissus pseudonarcissus* cultivar "Carlton", grown for the production of galanthamine, is mainly propagated vegetatively and therefore has low genetic variability (Hanks G. 2002, Narcissus and daffodil, the genus Narcissus, Taylor & Francis, London and New York, p. 53). Only the bulbs from this *Narcissus* cultivar are used as raw material for galanthamine production but they also contain significant quantities of haemanthamine and narciclasine (Phytochemistry 2013, 88: 43). Galanthamine, lycorine and lycorenine, along with eleven other alkaloids, were isolated from *Lycoris radiata* bulbs (Helv. Chim. Acta 2011, 94: 178), a plant cultivated in China as a galanthamine source.

The main disadvantages of the plants described in patent WO/2006/099635 as sources of galanthamine are their small size, short growing period and long period of physiological dormancy.

### Technical nature of the invention

The invention provides the composition of extracts in accordance with claim 1.

The composition of an extract from *Hippeastrum papilio,* the subject of this invention, may be part of a dry or liquid totally or partially purified extract, and obtained by a suitable extraction method and solvent.

The composition of extracts from *Hippeastrum papilio* according to claim 1 is obtained from selected whole plants or plant organs grown outdoors or in greenhouses in different geographical regions by conventional technologies, or as hydroponic cultures or by biotechnological methods. Feedstock is washed with clean water and allowed to dry in the air. After that the plants are cut mechanically to a size of 1-5 cm and subjected to drying in traditional equipment for drying fruits with circulating warm air. Drying is at a temperature of 15 ° to 85 ° C for 5 to 96 hours until the final moisture content of the biomass is less than 15%. The plant material is ground to a particle size from 85 microns to 3 mm using a conventional industrial plant mill.

To obtain the composition of the extracts from *Hippeastrum papilio* and the components thereof, the dry herbage is subjected to conventional procedures for liquid-liquid extraction of alkaloids. Dried and finely ground dry herbage containing between 0.05% and 2.0% of galanthamine in the dry herbage are loaded into a system of percolators and extracted with 0.1-5% (v) aqueous solution of an inorganic acid (eg. sulfuric, phosphoric, etc.) with countercurrent. The primary extract is made alkaline with 1-10% aqueous solution of potassium hydroxide or other bases to pH 8-9 and extracted with water-insoluble organic solvents such as chloroform, dichloromethane, *etc.* The organic solvent is washed with water and then alkaloids are extracted with 0.1-5% (v) aqueous solution of an inorganic acid. After basification of the aqueous solution with 1-10% aqueous solution of potassium hydroxide or other bases to pH 8-9, the composition of the extract from the *Hippeastrum papilio* and its components, the subject of the invention, is obtained by extraction with a water insoluble organic solvent such as chloroform, dichloromethane, hexane, benzene, ethyl acetate, etc. After evaporation of the solvent under vacuum, the alkaloids present in the extracts from *Hippeastrum papilio* are in the form of bases. They can be converted into pharmaceutically acceptable salts by the addition of a suitable acid before evaporation of the organic solvent. Other chemical or physical modification of the composition is allowed, such as derivatization (*eg.* esterification) or freezing to obtain by-products with altered physical and/or chemical properties.

Isolation and purification of the individual components of the extracts from *Hippeastrum papilio* can be achieved by chromatographic methods (such as preparative liquid chromatography, partitition chromatography, etc.).

The galanthamine and alkaloid composition mentioned above can be used in free form or in salt form, preferably in pharmaceutically acceptable salt form, for example, obtained by adding inorganic or organic acids, for example, hydrobromic acid. It can be also used after chemical or physical modifications.

Quality and quantity control of the plant material and the composition of the extracts from *Hippeastrum papilio* and the components thereof is performed by a method described in the literature (Strahil Berkov et al., Talanta 2011, vol. 83, no.5, pages 1455-1465). Dried plant material from the genus *Hippeastrum* (50 mg) is extracted with 1 ml of methanol containing 50 micrograms of codeine as an internal standard. The mixture is extracted at 60 ° C for 30 minutes and after centrifugation, 800 microliters of the supernatant are separated and evaporated. The resulting solids are dissolved in 500 microliters of 2% H₂SO₄ and extracted three times with 500 microliters of chloroform. Then the aqueous phase is made alkaline to pH 8-9 with 25% solution of ammonia and the alkaloids are extracted with chloroform. After evaporation of the organic solvent, the dry extract is dissolved in methanol prior to gas chromatography-mass spectrometry.

The composition of the extracts from *Hippeastrum papilio* and the components thereof are used in the production of drugs for the prevention or treatment of various diseases selected from mild to moderate Alzheimer's disease, myopathy, sensory and motor dysfunction, conditions associated with disorders of the central nervous system and others. For the above mentioned diseases and conditions, the required dose varies depending on the method of application and desired effect. Drugs are administered orally, for example, in the form of water-ethanol tinctures, tablets, coated tablets, capsules, oral sprays or sub-lingual agents, intranasal, in the form of nasal sprays, transdermal, or topical, for example, in the form of lotions, creams, ointments, skin patches or injectables. Preferably, the pharmaceuticals are in the form of a single dose. Each dose comprising the composition of extracts from *Hippeastrum papilio* or the components thereof may contain from 1.0 mg to 20 mg galanthamine.

The composition of the extracts from *Hippeastrum papilio* and the components thereof, the subject of this invention, are used in the manufacture of dietary supplements, which can be in the form of tablets, capsules or solutions. The composition of the extracts from *Hippeastrum papilio* and the components thereof are used in the production of dietary supplements for improving the quality of life of healthy people or to improve muscle strength and endurance.

The composition of the extracts from *Hippeastrum papilio* and the components thereof are also used in the production of pure substances of galanthamine, narwedine and haemanthamine, which are applied in the pharmaceutical industry and clinical research.

Pharmaceuticals and dietary supplements may contain from 0.01% to 99.9% of the composition of the extracts from the genus *Hippeastrum* and the components thereof. The other ingredients of the pharmaceuticals and dietary supplements can include plant metabolites (sugars, lipids, amino acids, organic acids, *etc.*), excipients such as lactose monohydrate, maltodextrin, water, ethanol or mixtures thereof.

The advantage of the composition of the extracts from *Hippeastrum papilio* and the various components thereof is that they are derived from selected, large and evergreen species and cultivars of the genus *Hippeastrum.* The extracts contain a high percentage of galanthamine and small amounts of narwedine, haemanthamine and other minor alkaloids, which facilitates the purification of galanthamine. The other alkaloids have low toxicity or no toxicity, allowing the composition of the extracts from *Hippeastrum papilio* to be used for the production of pharmaceuticals and dietary supplements. The composition of the extracts from *Hippeastrum papilio* and the components thereof can be used as a source for the industrial production of galanthamine and haemanthamine.

### Figure captions

Fig. 1, GC-MS chromatogram of the composition of an extract from unselected plants of *Hippeastrum*, in which the three main alkaloids of the composition are indicated;
Fig. 2, GC-MS chromatogram of composition of an extract from unselected plants of the genus *Hippeastrum*, in which the three main alkaloids of composition are indicated;
Fig. 3, GC-MS chromatogram of the composition of an extract from unselected plants of the genus *Hippeastrum*;
Fig. 4, GC-MS chromatogram of the composition of an extract from selected plants of the genus *Hippeastrum*, in which the three main alkaloids are indicated;
Fig. 5, GC-MS metabolic profile of a total methanol extract from the genus *Hippeastrum.*

**The invention is illustrated by the following examples of performance without limiting it**

### Example 1

The invention provides the composition of an extract from the genus *Hippeastrum* containing the following components measured by GC-MS: galanthamine 54.1%, narwedine 1.7%, haemanthamine 19.5%, an alkaloid of homolycorine type 13.6%, other alkaloids 10.7% and non-alkaloid metabolites 0.4% (Table 1).

Five grams of the raw material - powdered dry herbage from unselected plants of the species *Hippeastrum papilio*, were extracted three times for 24 hours with 40 ml of 2% aqueous solution of sulfuric acid (H₂SO₄). The aqueous solutions were combined and made alkaline with ammonia to pH 8-9, and then the resulting aqueous fraction was extracted twice with 30 ml of chloroform. The combined chloroform fraction was washed with 20 ml of water and then the organic solvent was extracted three times with 20 ml of 2% aqueous solution of H₂SO₄. The alkaloids were obtained from the aqueous phase after basification with ammonia to pH 8-9 and two-time extraction with 20 ml of chloroform. After evaporation of the organic solvent the dry composition of extract from the genus *Hippeastrum* was dissolved in methanol before GC-MS analysis.

Quality and quantity control of the plant material and the composition of extract from the genus *Hippeastrum* is performed by a method described above by GC-MS.

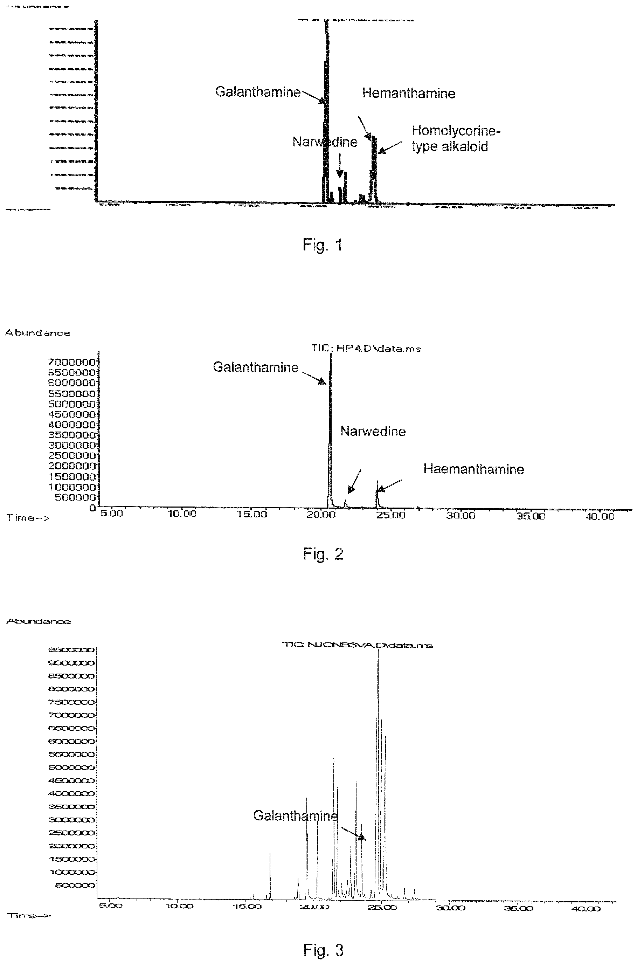

Data from the analysis are presented in Fig. 1 and Table 1.

| Table 1 | | | | | | |
|---|---|---|---|---|---|---|
| Examples of the composition of extracts from the genus *Hippeastrum* and the components thereof derived from plants of different origins. The content of the main alkaloids in the composition is expressed as a percentage based on GC-MS analyses. | | | | | | |
| | Plant (code) | Galanthamine % | Narwedine % | Hemanthamine % | Other alkaloids % | Non-alkaloids |
| *Unselected plants* | | | | | | |
| 1 | *H. papilio* (PAP) | 54.1 | 1.7 | 19.5 | 24.3 | 0.4 |
| 2 | *H. papilio* (HPB) | 62.5 | 5.9 | 22.5 | 8.0 | 0.1 |
| 3 | *H. papilio* (HBR) | 88.4 | 1.3 | 9.6 | 0.7 | Not detected |
| 4 | *H. papilio* (HRE) | 83.2 | 3.8 | 8.3 | 4.6 | 0.1 |
| 5 | *H. vittatum* (HIV) | 4.6 | 0.1 | 0.9 | 93.6 | 0.8 |

| *Selected plants* | | | | | | |
|---|---|---|---|---|---|---|
| 5 | *H. papilio* (HP1) | 94.3 | 1.5 | 3.7 | 0.4 | 0.1 |
| 6 | *H. papilio* (HP2) | 90.1 | 1.7 | 4.1 | 3.4 | 0.7 |
| 7 | *H. papilio* (HP13) | 95.6 | 1.2 | 1.9 | 1.3 | Not detected |
| 8 | *H. papilio* (HP14) | 97.8 | 0.7 | 0.8 | 0.3 | 0,4 |
| 9 | *H. papilio* (HP17) | 91.4 | 1.7 | 6.4 | 0.3 | 0.2 |

### Example 2.

Prepared as in Example 1, but with unselected plants of *Hippeastrum papillio* from a different origin than in Example 1. The invention provides a composition of an extract from *Hippeastrum papillio* and its components, which include the following: galanthamine 88.4%, narwedine 1.3%, haemanthamine 9.6%, and other alkaloids 0.7%.

Data from the analysis are presented in Fig. 2 and Table 1.

### Comparative Example 3

Prepared as in Examples 1 and 2, but with unselected plants from *Hippeastrum vittatum*, whose extract contains the following components: galanthamine 4.6%, narwedin 0.1%, haemanthamine 0.9%, other alkaloids 93.6% and non alkaloid compounds 0.8%.

The results are presented in Fig. 3 and Table 1.

Table 1 presents other results from the analysis of the composition of extracts from unselected plants of the genus *Hippeastrum*, like Examples 1, 2 and comparative example 3. These results show qualitative and quantitative variation in the alkaloid profiles, respectively, in the composition of extracts from the genus *Hippeastrum* obtained from unselected plants and plants of different origin. This variability allows appropriate selection of genotypes from different species and of different origin within the species with desirable properties and extract composition.

### Example 4.

Prepared as in Examples 1, 2 and comparative example 3, but with selected plants of *Hippeastrum papillio*, whose extract contains the following components: galanthamine 97.8%, narwedine 0.7%, haemanthamine 0.8%, other alkaloids 0.3%, and non alkaloid compounds 0.4%.

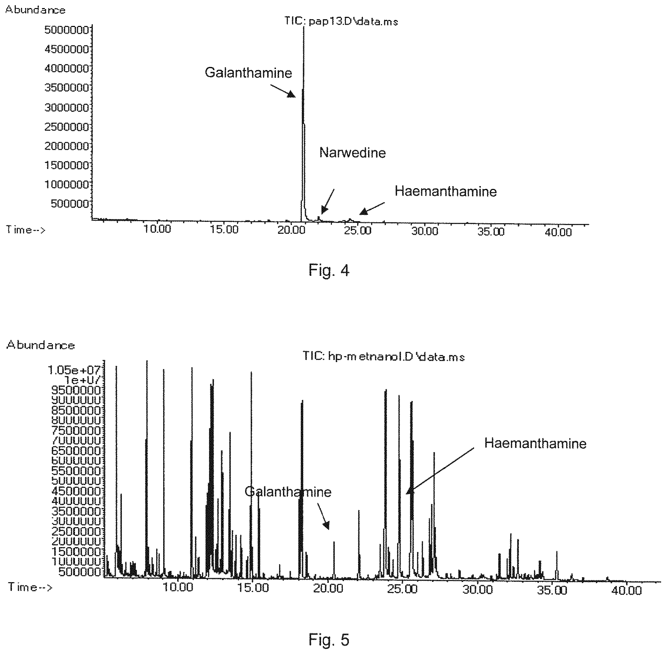

The results are presented in Fig. 4 and Table 1.

Table 1 presents the results of other analyses of selected plants, as in Example 3. These results show the potential of selected plants to provide an extract composition from the genus *Hippeastrum,* whose components have certain characteristics, namely high levels of galanthamine.

### Example 5.

The invention provides a total alcoholic extract, which is used to obtain the composition of an extract from the genus *Hippeastrum* and its components.

Five grams of powdered plant material is macerated in 35 ml of methanol for 48 hours at room temperature. After filtration under pressure, the plant material is washed with two portions of 15 ml of methanol, which are combined with the filtrate in a total methanol extract.

Aliquots of 50 microliters of the resulting total extract is transferred to gas chromatography vials and evaporated in a vacuum evaporator. The dry extract is dissolved in pyridine (50 microliter) and derivatized for 90 min at 60°C with 50 microliters of BSTFA (*N*,*O*-bis(trimethylsilyl)trifluoroacetamide). The derivatized extracts are diluted with 300 microliters of chloroform before analysis with GC-MS.

The rest of the total methanol extract is concentrated under vacuum to give a total dry extract. This extract is dissolved in 30 ml of 2% aqueous solution of H₂SO₄ and extracted three times with 15 ml of chloroform. Then, the aqueous solution is made alkaline with ammonia to pH 8-9 and the alkaloids are extracted twice with 20 ml of chloroform. The combined chloroform extracts are evaporated under vacuum to obtain a dry alkaloid fraction representing the composition of the extract from the genus *Hippeastrum.* The alkaloid profiles from GC-MS analyses are similar to those from Examples 1 to 4, depending on the origin of the sample.

Metabolic profile of the resulting total methanol extract is shown in Fig. 5.

## Claims

1. Composition of extracts from *Hippeastrum papilio* comprising galanthamine, narwedine and haemanthamine obtainable by conventional procedures for liquid-liquid extraction of alkaloids, and **characterized in that** it contains the following components as determined by integration of their GC-MS peak areas and expressed as a percentage of their total ion current: galanthamine from 40% to 98%, haemanthamine from 0.01% to 35%, narwedine from 0.01% to 10%, and other alkaloids of homolycorine-, and/or tazettine-, and/or monthanine- and/or lycorine-type from 0.001% to 35%, and non-alkaloid plant metabolites from 0.001 to 1 %, and **in that** it is derived from the total extracts from dry herbage of *Hippeastrum papilio.*

2. Composition of extracts according to claim 1, which is in dry or liquid form.

3. Composition of extracts according to claim 1, of which the alkaloids are in free form or in the form of pharmaceutically acceptable salts.

4. Use of the composition of extracts according to claims 1 to 3 for isolation of pure compounds of haemanthamine and galanthamine and galanthamine in particular.

5. Composition of extracts according to Claims 1 to 3, for use in treatment of diseases or conditions selected from mild to moderate vascular dementia, Alzheimer's disease, myopathy, sensory and motor dysfunction of the central nervous systems.

6. Use of the composition of extracts according to Claims 1 to 3, for the production of pharmaceuticals and dietary supplements that include components from the composition of the extracts as pure substances.

## Patentansprüche

1. Zusammensetzung von Extrakten von *Hippeastrum papilio,* umfassend Galanthamin, Narwedin und Haemanthamin, erhältlich durch konventionelle Verfahren für die Flüssig/Flüssig-Extraktion von Alkaloiden, und **gekennzeichnet dadurch, dass** sie die folgenden Bestandteile enthält wie bestimmt durch Integration von deren GC-MS-Peak-Flächen und ausgedrückt als Prozentsatz von deren Totalionenstrom: Galanthamin von 40% bis 98%, Haemanthamin von 0,01% bis 35%, Narwedin von 0,01% bis 10%, und andere Alkaloide vom Homolycorin- und/oder Tazettin- und/oder Monthanin- und/oder Lycorin-Typ von 0,001% bis 35%, und Nichtalkaloid-Pflanzenmetabolite von 0,001% bis 1%, und dadurch, dass sie abgeleitet ist von den Gesamtextrakten getrockneter Gräser von *Hippeastrum papilio*.

2. Zusammensetzung von Extrakten nach Anspruch 1, welche in trockener oder flüssiger Form ist.

3. Zusammensetzung von Extrakten nach Anspruch 1, von der die Alkaloide in freier Form oder in Form ihrer pharmazeutisch annehmbaren Salze sind.

4. Verwendung der Zusammensetzung von Extrakten gemäß den Ansprüchen 1 bis 3 zur Isolation von reinen Verbindungen von Haemanthamin und Galanthamin, und Galanthamin insbesondere.

5. Zusammensetzung von Extrakten gemäß den Ansprüchen 1 bis 3 zur Verwendung in der Behandlung von Erkrankungen oder Zuständen, ausgewählt aus milder bis moderater vaskulärer Demenz, Alzheimer-Krankheit, Myopathie, sensorischer und motorischer Dysfunktion des zentralen Nervensystems.

6. Verwendung der Zusammensetzung von Extrakten gemäß den Ansprüchen 1 bis 3 zur Herstellung von Pharmazeutika und Nahrungsergänzungsmitteln, die Bestandteile der Zusammensetzung von Extrakten als Reinsubstanzen einschließen.

## Revendications

1. Composition d'extraits de *Hippeastrum papilio* comprenant de la galanthamine, de la narwédine et de l'hémanthamine, pouvant être obtenus par des procédures conventionnelles pour l'extraction liquide-liquide d'alcaloïdes, **caractérisée en ce qu'**elle contient les composants suivants, tels que déterminés par intégration de leurs surfaces de pics CG-SM et exprimés en pourcentage de leur courant ionique total : galanthamine de 40 % à 98 %, hémanthamine de 0,01 % à 35 %, narwédine de 0,01 % à 10 %, et autres alcaloïdes de type homolycorine et/ou tazettine et/ou monthanine et/ou lycorine de 0,001 % à 35 %, et métabolites végétaux non alcaloïdes de 0,001 à 1 %, et **en ce qu'**elle est dérivée des extraits totaux d'herbes sèches de *Hippeastrum papilio.*

2. Composition d'extraits selon la revendication 1, qui est sous forme sèche ou liquide.

3. Composition d'extraits selon la revendication 1, dont les alcaloïdes sont sous forme libre ou sous la forme de sels pharmaceutiquement acceptables.

4. Utilisation de la composition d'extraits selon les revendications 1 à 3 pour l'isolation de composés purs d'hémanthamine et de galanthamine et de galanthamine en particulier.

5. Composition d'extraits selon les revendications 1 à 3, pour une utilisation dans le traitement de maladies ou d'états choisis parmi une démence vasculaire légère à modérée, la maladie d'Alzheimer, une myopathie, un dysfonctionnement sensoriel et moteur du système nerveux central.

6. Utilisation de la composition d'extraits selon les revendications 1 à 3, pour la production de produits pharmaceutiques et de compléments alimentaires qui comprennent des composants de la composition d'extraits sous la forme de substances pures.
